# EUROPEAN PATENT APPLICATION

(11) **EP 4 599 789 A1**
(43) Date of publication of application: **13.08.2025**
(21) Application number: 23888948.9
(22) Date of filing: 13.10.2023
(51) Int. Cl.: A61B 90/00, A61B 34/10, G06T 7/30

(54) **IMAGE REGISTRATION-BASED MEDICAL IMAGE GENERATION DEVICE USING AUGMENTED REALITY, AND METHOD OF OPERATING SAME**

(30) Priority: 10.11.2022 KR 20220149309
(71) Applicant: The Asan Foundation, Seoul 05505 (KR); UNIVERSITY OF ULSAN FOUNDATION FOR INDUSTRY COOPERATION, Nam-gu Ulsan 44610 (KR); Skia, Seoul, 08390 (KR)
(72) Inventor: CHOI, Jong Woo, Seoul 06080 (KR); NA, Seungwon, Yongin-si, Gyeonggi-do 17117 (KR); JEONG, Woo Shik, Seoul 06622 (KR); KIM, Young Chul, Seoul 05698 (KR); PARK, Chan Ung, Seoul 08768 (KR); KWON, Hyuk, Seoul 08208 (KR); LEE, Jong Myoung, Seoul 06658 (KR)
(74) Representative: dompatent von Kreisler Selting Werner - Partnerschaft von Patent- und Rechtsanwälten mbB
(86) International application number: PCT/KR2023/015812
(87) International publication number: WO 2024/101685

(57) **Abstract**

Disclosed is an operating method of a medical image processing device using augmented reality (AR) including obtaining, from a first external device, a first image including blood vessel information and bone tissue information according to tomography, obtaining, from a second external device, a second image including a facial structure and a facial curve according to 3D imaging, extracting a facial feature point from facial unique information including the blood vessel information, the bone tissue information, the facial structure, and the facial curve, registering the first image to the second image based on the facial feature point, generating a composite image by performing 3D-modeling on the first image registered with the second image, projecting the composite image onto a face of a patient, and displaying, on a projected image, a first layer corresponding to the blood vessel information and a second layer including the bone tissue information.

## Description

### [TECHNICAL FIELD]

Embodiments of the present disclosure described herein relate to a medical image generation device and an operating method thereof, and more particularly, relate to a medical image generation device based on image registration using augmented reality and an operating method thereof.

### [BACKGROUND ART]

Augmented Reality (AR) is a technology that overlaps a three-dimensional (3D) virtual image onto a real image or background to display the overlapped image as a single image. Nowadays, there are attempts to apply the AR technology to a medical field.

As a conventional technology, there are AR-based navigation technologies that simply visualize, through the AR technology, the results of rigid registration by using images (CT/MRI) captured before surgery. The AR navigation technology requires the registration of a coordinate system between a patient's body and 3D objects. Accordingly, a conventional AR navigation technology performs the registration of the coordinate system by utilizing markers as information from a real world.

Marker-based AR requires precise installation of markers because the registration of medical images is determined based on only markers. Accordingly, there is a need for a new technology capable of suppressing the occurrence of human error in all processes of using augmented reality, either while markers are not installed, or while relative locations of markers to the patient's body are maintained after markers are installed.

### [DETAILED DESCRIPTION OF THE INVENTION]

### [TECHINICAL PROBLEM]

Embodiments of the present disclosure provide a medical image processing device using AR and an operating method thereof.

Problems to be solved by the present disclosure are not limited to the problems mentioned above, and other problems not mentioned will be clearly understood by those skilled in the art from the following description.

### [TECHNICAL SOLUTION]

According to an embodiment, an operating method of a medical image processing device using augmented reality (AR) includes obtaining, from a first external device, a first image including blood vessel information and bone tissue information according to tomography, obtaining, from a second external device, a second image including a facial structure and a facial curve according to 3D imaging, extracting a facial feature point from facial unique information including the blood vessel information, the bone tissue information, the facial structure, and the facial curve, registering the first image to the second image based on the facial feature point, generating a composite image by performing 3D-modeling on the first image registered with the second image, projecting the composite image onto a face of a patient, and displaying, on a projected image, a first layer corresponding to the blood vessel information and a second layer including the bone tissue information.

According to an embodiment, a medical image processing device using AR includes a memory that stores a feature extraction module configured to extract a feature part from an image, an image registration module that registers at least two images, an image processing module that performs image processing on data constituting an image according to a predefined operation, and an AR processing module that implements and displays the processed image in augmented reality, and a processor including one or more cores and controlling operations of the feature extraction module, the image registration module, the image processing module, and the AR processing module. The memory obtains a first image including blood vessel information and bone tissue information, and a second image including a facial structure and a facial curve from an external device. The processor extracts a facial feature point from facial unique information including the blood vessel information, the bone tissue information, the facial structure, and the facial curve, registers the first image to the second image based on the facial feature point, generates a composite image by performing 3D-modeling on the first image registered with the second image, projects the composite image onto a face of a patient, and displays, on a projected image, a first layer corresponding to the blood vessel information and a second layer including the bone tissue information.

Besides, a computer program stored in a computer-readable recording medium for execution to implement the present disclosure may be further provided. According to an embodiment, when executed by one or more processors, a computer program stored in a computer-readable storage medium performs the following operations for performing an operating method of a medical image processing device using AR. The operations includes extracting a facial feature point from facial unique information including blood vessel information, bone tissue information, a facial structure, and a facial curve, which are obtained by an external device, registering the first image to the second image based on the facial feature point, generating a composite image by performing 3D-modeling on the first image registered with the second image, projecting the composite image onto a face of a patient, and displaying, on a projected image, a first layer corresponding to the blood vessel information and a second layer including the bone tissue information.

In addition, a computer-readable recording medium for recording a computer program for performing the method for implementing the present disclosure may be further provided.

### [ ADVANTAGEOUS EFFECTS OF THE INVENTION]

According to the above-mentioned problem solving means of the present disclosure, a facial AR technology may be provided to minimize errors in consideration of the complex and delicate structure of a facial area through AR technology centered on the facial area.

According to the above-mentioned problem solving means of the present disclosure, a device, a method, and a computer program for processing a medical image based on augmented reality that may improve the accuracy of coordinate system registration through non-marker-based coordinate system registration may be provided.

According to the above-mentioned problem solving means of the present disclosure, the occurrence of human error may be suppressed in all processes of using augmented reality, either while there is no need to install markers, or while relative locations of markers to a patient's body are maintained after markers are installed.

According to the present disclosure, an image registered with a CT image in the 3D image may be projected onto a patient's facial area, and thus the location and depth of each of a bone tissue and a blood vessel tissue placed within the facial area may be visually displayed, thereby achieving the accurate and safe surgery of the facial area.

Effects of the present disclosure are not limited to the effects mentioned above, and other effects not mentioned will be clearly understood by those skilled in the art from the following description.

### [ DESCRIPTION OF THE DRAWINGS]

FIG. 1 is a block diagram illustrating a medical image processing system, according to an embodiment of the present disclosure.
FIG. 2 is a flowchart illustrating an operating method of a medical image processing device, according to an embodiment of the present disclosure.
FIG. 3 is a detailed flowchart illustrating an operating method of a medical image processing device, according to an embodiment of the present disclosure.
FIG. 4 is a detailed flowchart illustrating an operating method of a medical image processing device, according to an embodiment of the present disclosure.
FIG. 5 is a diagram illustrating the synthesis of a tomography image and a 3D image according to an image registration operation of a medical image processing device, according to an embodiment of the present disclosure.
FIG. 6 is a diagram showing a projection of a bone tissue layer according to an image synthesis operation of a medical image processing device, according to an embodiment of the present disclosure.
FIG. 7 is a diagram showing a projection of a blood vessel layer according to an image synthesis operation of a medical image processing device, according to an embodiment of the present disclosure.

### [ BEST MODE]

The same reference numerals denote the same elements throughout the present disclosure. The present disclosure does not describe all elements of embodiments. Well-known content in a technical field to which the present disclosure belongs or redundant content in which embodiments are the same as one another will be omitted. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, a 'medical image processing device' includes all various devices capable of providing results to a user by performing arithmetic processing. For example, the medical image processing device according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

A server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

Augmented Reality (AR) is a technology that overlaps a three-dimensional (3D) virtual image onto a real image or background to display the overlapped image as a single image. This AR technology is being applied to various fields such as games, health, and map services through various smart devices. Nowadays, the AR technology is being used in a medical field.

Surgeries where the benefits of augmented reality are emphasized are a head and neck surgery and a facial surgery. Augmented reality may be used in surgeries which require precise anatomical information due to the complex and delicate structures of a head and neck area and a facial area, thereby allowing medical staff to intuitively identify medical images. In particular, because the facial area has clear and numerous anatomical landmarks compared to other body parts, the AR technology may be very helpful in determining locations of surgical targets and distances from major landmarks.

The most commonly used AR implementing method in the medical field refers to a 'marker-based AR' method requiring the installation of fiducial markers, which serve as the basis for medical image projection, on a rigid body. In orthopedic surgeries, bones may be often surgical targets, and thus markers may be installed in the bones.

However, in head and neck surgeries and facial surgeries, soft tissues such as the brain or skin are often targets, and in the case of facial fracture surgeries, it is difficult to install markers on a skull. Even when a marker is installed on a forehead where bones and epidermis are close and there is little fat layer, there is room for errors to occur due to human error during a process of installing markers. Marker-based AR requires precise installation of markers because the registration of medical images is determined based on only markers. Accordingly, there is a need for a new technology capable of suppressing the occurrence of human error in all processes of using augmented reality, either while markers are not installed, or while relative locations of markers to the patient's body are maintained after markers are installed.

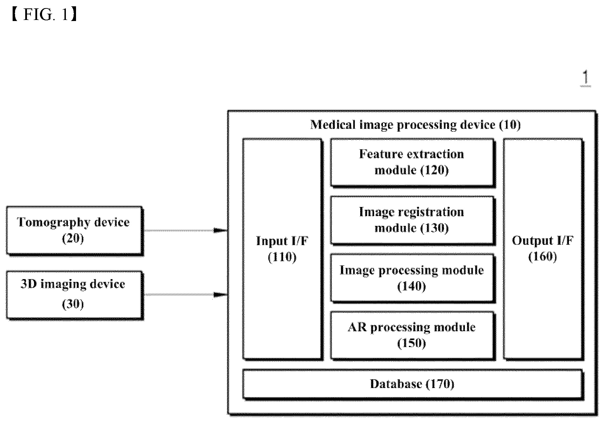

FIG. 1 is a block diagram illustrating a medical image processing system 1, according to an embodiment of the present disclosure.

Referring to FIG. 1, the medical image processing system 1 may include a medical image processing device 10, a tomography device 20, and a 3D imaging device 30.

The medical image processing system 1 may obtain medical information of a patient prior to a patient's surgery or procedure, may extract pieces of feature data necessary for the procedure from the obtained medical information, and may process the pieces of feature data into data necessary for the surgery or procedure. According to an embodiment, the tomography device 20 may process the internal anatomical plane of a human body as images by reconstructing the results of x-rays or ultrasound of the human body through computed tomography (CT). In the present disclosure, CT imaging is described by means of example for convenience of description. However, the tomography device 20 may refer to capturing images of the inside of the human body through computed tomography (CT), magnetic resonance imaging (MRI), and positron emission tomography (PET).

According to an embodiment, the 3D imaging device 30 may obtain 3D image information about an object by using a stereo camera and/or a depth camera.

According to an embodiment of the present disclosure, the medical image processing system 1 may perform marker-less-based medical image processing through data analysis of the medical image processing device 10, which obtains a tomography image from the tomography device 20 and a 3D image from the 3D imaging device 30, without physically installing an artificial marker for extracting a medical image. Accordingly, the medical image processing system 1 may suppress the occurrence of human error in all processes of using augmented reality, either while there is no need to install markers, or while relative locations of markers to a patient's body are maintained after markers are installed.

In an embodiment of the present disclosure, the medical image processing system 1 may project a marker-less-based medical image onto the patient's face by using AR technology. Accordingly, the medical image processing system 1 may visually display the location and depth of each of a bone tissue and a blood vessel tissue located within a facial area, and medical staff may perform an accurate and safe facial surgery by using the medical image processing system 1.

The medical image processing device 10 may include an input interface (I/F) 110, a feature extraction module 120, an image registration module 130, an image processing module 140, an augmented reality (AR) processing module 150, an output I/F 160, and a database 170. The medical image processing device 10 of FIG. 1 is only an embodiment of the present disclosure, and thus the present disclosure is not limited to FIG. 1.

The input I/F 110 may be used to enter image information (or signal), audio information (or signal), data, or information entered by a user. The input I/F 110 may include at least one of at least one camera, at least one microphone, and the user input I/F 110. Speech data or image data collected by the input I/F 110 may be analyzed and processed as a control command of a user.

In an embodiment of the present disclosure, the input I/F 110 serves as a passage for various types of external devices connected to the present device. The interface unit may include at least one of a wired/wireless headset port, an external charger port, a wired/wireless data port, a memory card port, a port for connecting a device equipped with a subscriber identification module (SIM), an audio input/output (I/O) port, a video I/O port, and an earphone port. In the present device, appropriate control related to an external device connected to the interface unit may be performed.

The camera may process an image frame such as a still image or a moving image, which is obtained by an image sensor in a shooting mode. The processed image frame may be displayed through a display unit, may be directly projected as an optical signal onto the patient's facial area, or may be stored in a memory.

In the meantime, when there are a plurality of cameras, the plurality of cameras may be arranged to form a matrix structure. In this way, pieces of image information having various angles or focuses may be input through the cameras forming a matrix structure. Furthermore, the cameras may be arranged in a stereo structure to obtain left and right images for implementing a three-dimensional stereoscopic image.

The user input I/F 110 may be used to receive information from a user. When information is entered through the user input I/F 110, a control unit may control operations of the present device to correspond to the input information. This user input I/F 110 may include a hardware-type physical key (e.g., a button, a dome switch, a jog wheel, or a jog switch that is located on at least one of the front, back, and sides of the present device) and a software-type touch key. For example, the touch key may consist of a virtual key, a soft key, or a visual key displayed on a touch screen-type display unit through software processing or may consist of a touch key positioned on a portion other than the touch screen. In the meantime, the virtual key or the visual key may be displayed on the touch screen while having various shapes. For example, the virtual key or the visual key may be formed of graphics, texts, icons, video, or a combination thereof.

The sensor unit senses at least one of information about interiors of the present device, surrounding environmental information surrounding the present device, and user information and generates a sensing signal corresponding to the sensed result. On the basis of the sensing signal, the control unit may control the driving or operation of the present device or may perform the data processing, function, or operation associated with an application program installed in the present device.

The sensor unit may include at least one of a proximity sensor, an illumination sensor, a touch sensor, an acceleration sensor, a magnetic sensor, a G-sensor, a gyroscope sensor, a motion sensor, an RGB sensor, an infrared sensor (IR sensor), a finger scan sensor, an ultrasonic sensor, an optical sensor (e.g., a camera), a microphone, an environmental sensor (e.g., including at least one of a barometer, a hygrometer, a thermometer, a radiation detection sensor, a heat detection sensor, and a gas detection sensor), and a chemical sensor (e.g., a healthcare sensor, a biometric sensor, or the like). In the meantime, the present device may combine and utilize pieces of information sensed by at least two or more of these sensors.

According to an embodiment of the present disclosure, the input I/F 110 may receive a medical information image of the patient's face from an external device. For example, a camera among external devices may obtain real-time images including information about the space where the patient is placed. For example, the camera may obtain real-time images of the patient's facial area. For example, the camera may extract at least one feature point from a facial image and continuously calculate the location and direction of a depth camera. The location and direction of the camera may be used to track AR-based medical images as described below.

The feature extraction module 120 may be configured to extract a feature part from an image. According to an embodiment, the feature extraction module 120 may extract feature points from body tissues or body parts, which are features or references, such as blood vessels, bone tissues, facial curves, and various bones, in a medical image. In the present disclosure, information of a feature point may include 3D location information in a 3D space, and the 3D location information of the feature point may function as a marker.

The image registration module 130 may be configured to register at least two images. According to an embodiment, the image registration module 130 may register (or match) at least one feature point extracted from two or more images. The image registration module 130 may use a widely known image registration algorithm or store a lookup table required for stereo conversion for image registration in advance and may load the calculation results whenever necessary. According to an embodiment, the image registration module 130 may perform downsize-sampling on a first feature point, may select a point pair, quantizes a location and a direction (i.e., a location and a normal vector) by using a difference in the point pair so as to be indexed, and may store the indexed result in the lookup table.

According to an embodiment of the present disclosure, the image registration module 130 may register a 3D image and a tomography image based on feature points. Accordingly, the image registration module 130 registers marker-less-based coordinate systems that do not use artificial markers, and specifically, registers coordinate systems by setting feature points within a face to function as markers.

According to an embodiment of the present disclosure, the image registration module 130 may continuously calculate the registration between the composite image and the facial feature points depending on the change in a real-time patient facial image. Generally, the patient is under anesthesia during a surgery or a procedure, and thus a change is not great. However, depending on the surgery or procedure performed by the medical staff, the location and the angle may be slightly changed as the facial area is touched by hands or medical instruments. The image registration module 130 may achieve accurate image projection by continuously calculating the registration between the composite image and the facial feature points depending on the change in the real-time patient facial image.

In an embodiment, the image registration module 130 may perform primary registration on the composite image with the real-time patient face image based on portions corresponding to locations of eyes. In an embodiment, the image registration module 130 may perform the primary registration to increase the accuracy of registration, and then perform second registration on the composite image and the real-time patient face image based on portions corresponding to the face other than the locations of eyes. That is, the image registration module 130 may increase the accuracy of registration by first performing primary registration based on information about reference points (e.g., both eyes) relatively less likely to be deformed.

In an embodiment, the image registration module 130 may perform registration by using a first registration algorithm and a second registration algorithm. For example, the image registration module 130 may calculate approximate locations of a first feature point from a first image including facial CT-based blood vessels and bone tissues and a second feature point from a second image based on facial 3D imaging based on the first registration algorithm. Here, the approximate locations may mean locations and directions.

The image registration module 130 may calculate the approximate locations of the first feature point and the second feature point by using, for example, a point pair feature (PPF) algorithm as the first registration algorithm. In particular, the image registration module 130 may select a point pair after performing downsize-sampling on feature points. Afterwards, the image registration module 130 may quantize the selected point pair (pieces of information, which are not continuous but belong to a specific section, is merged into a piece of information) to select the identical or similar point pair from the lookup table. For example, the image registration module 130 may select a candidate group obtaining the highest score in similarity of the quantized point pair and may calculate the approximate locations of the first feature point and the second feature point based on this. In an embodiment, the PPF algorithm uses quantization (pieces of information, which are not continuous but belong to a specific section, is merged into a piece of information), and thus requires accuracy improvement. Accordingly, in the present disclosure, the accuracy of registration is increased by additionally using the second algorithm described below together with the PPF algorithm.

The image registration module 130 may calculate the approximate locations of the first feature point and the second feature point, and then may calculate precise locations of a plurality of first feature points corresponding to reference skin and a plurality of second feature points corresponding to the reference skin based on the second registration algorithm. The image registration module 130 may calculate precise locations of the plurality of first feature points and the plurality of second feature points by using, for example, an iterative closest point (ICP) algorithm as the second registration algorithm that takes the above-described approximate locations as starting points. Here, the precise locations may mean movement and rotation.

That is, the image registration module 130 detects a location and a direction by using the approximate locations through the PPF algorithm, and detects movement and rotation by using the precise locations through the ICP algorithm so as to make the distance between points closer. In this way, the present disclosure may search for an approximate location by using the PPF algorithm and may perform registration through the ICP algorithm by using the approximate location as a starting point, thereby increasing the accuracy of the registration.

In another embodiment, the image registration module 130 may perform registration by using only the second registration algorithm. For example, the image registration module 130 may set an initial value by performing pre-registration at a plurality of predetermined points. Here, the pre-registration may be performed through the ICP algorithm. The image registration module 130 may perform pre-registration, for example, at the center of the upper surface of a scene bounding box corresponding to a real-time image and the center of a second scene bounding box. Afterwards, the image registration module 130 may set, as initial values, the location and direction of a point having the minimum residual error among the results of the pre-registration. Next, the image registration module 130 may perform registration by calculating precise locations of the plurality of first feature points and the plurality of second feature points by applying the ICP algorithm from initial values.

The image processing module 140 may perform image processing configured to perform image processing on data constituting an image depending on predefined operations.

In an embodiment, the image processing module 140 may remove noise from a medical image by using an anisotropic diffusion filter (ADF). Unlike noise removal filters thus commonly used, the ADF analyzes information of an image to effectively remove noise while maintaining edge information. The noise may be removed from a medical image, thereby minimizing errors in facial feature point information due to noise when feature points are extracted from a facial image.

In an embodiment, the image processing module 140 may distinguish between a background and a patient area in a medical image by using Otsu's Method. The Otsu's method is a technique that automatically finds an adaptive threshold value for an input image to segment regions by analyzing the brightness distribution of the input image. A thresholding technique using the Otsu's method refers to a technique that only uses the brightness value of an image and does not consider the topology of the image. Accordingly, the image processing module 140 may sequentially perform a seeded region growing (SRG) operation and a morphology operation in consideration of topology information of the image to remove the mis-segmented region. The SRG refers to a technique that uses a process of tracking, expanding, and finding adjacent pixels satisfying conditions based on a seed. The morphology operation removes mis-segmented noise and empty space from the segmentation results by repeatedly performing erosion and dilation.

In an embodiment, the image processing module 140 may segment a patient region on a slice-by-slice basis of a medical image. The entire segmented image is used as volume data for generating a skin mesh. In an embodiment, a part of the medical image may be 3D modeled data in the form of the skin mesh. For example, the 3D modeled medical image may be 3D mesh information including 3D vertex information, connection information thereof, and surface information generated thereby.

In an embodiment, the image processing module 140 may generate a skin mesh 205 by using a marching cube technique. The image processing module 140 may smooth volume data by applying a Gaussian filter to a 3D image when generating the skin mesh. For this reason, an effect similar to applying smoothing to a mesh to be generated through a marching cube may be achieved.

The image processing module 140 may perform mesh simplification using a Fast-Quadric Mesh Simplification algorithm. It is possible to reduce an amount of computation and a memory by performing mesh simplification.

The AR processing module 150 may be configured to implement and display the processed image as augmented reality.

The AR processing module 150 may generate an AR-based medical image for outputting medical information on the patient's body (in particular, a face) in a real-time image based on the patient's facial feature points.

In an embodiment, the AR processing module 150 may adjust the transparency and contrast of the patient's body modeling in a real-time image such that a doctor identifies the accuracy of registration. Moreover, brightness may be adjusted to more easily identify lesions and tissues of interest in the real-time image.

In an embodiment, the AR processing module 150 may output, as medical information on the patient's body in the real-time image, at least one of the following: a CT plane, a lesion, location information of a skin closest to the lesion, location information of the lesion and the skin in an anatomical axis direction, and a scale for measuring a distance between the lesion and the skin.

In an embodiment of the present disclosure, the AR processing module 150 may separate and display a first layer corresponding to blood vessel information, and a second layer including bone tissue information on a projected image projected on the patient's face. For example, when the blood vessel information as the first layer is projected onto the patient's face in an AR method, the bone tissue information corresponding to the second layer may be displayed separately at a point in time when the first layer is not displayed, or in an area where it is not displayed. According to an embodiment, the first layer and the second layer may be displayed simultaneously.

In an embodiment of the present disclosure, when a doctor selects a specific CT slice, the AR processing module 150 may place a specific CT slice 401 at a location (inside the patient's torso) corresponding to a he location of the corresponding CT slice on the patient's body in a real-time image.

In an embodiment of the present disclosure, the AR processing module 150 may stack the CT slices 401 to form data in three dimensions, may generate an anatomical plane image in a desired direction from the data, and may express the anatomical plane image.

In an embodiment of the present disclosure, the AR processing module 150 may change and express the first color (e.g., red) of the lesion area in the anatomical plane image.

In an embodiment of the present disclosure, the AR processing module 150 may express lines, shapes, text, or the like on a patient's body in a real-time image depending on the doctor's input.

In an embodiment of the present disclosure, when the doctor enters lines, shapes, text, or the like on the patient's body in the real-time image, the AR processing module 150 may adjust the transparency of at least one element (a disk for identifying a lesion, a size, and a location) displayed on a screen in real time. For this reason, the doctor may easily identify lines, shapes, text, or the like on the patient's body in the real-time image.

In an embodiment of the present disclosure, the AR processing module 150 may continuously output AR-based medical images by tracking spatial information based on the location and direction of a depth camera. In other words, the AR processing module 150 may continuously maintain the registration between 3D modeled data and a real-time image, by registering 3D modeled medical image data and the real-time image at least once, and then tracking spatial information of each frame by using a third algorithm. Here, the third algorithm may be a simultaneous localization and mapping (SLAM) algorithm. In the meantime, the real-time image may be registered at each frame for real-time tracking (detecting changes in the location and direction of a depth camera described later). Accordingly, issues indicating that the amount of computation according to the registration between the 3D modeling data and the real-time image is increased for each frame may be solved.

In an embodiment of the present disclosure, the AR processing module 150 may organize space into 3D data based on image information (RGB information or depth information) of the space, and may determine the location and direction of the depth camera from the 3D data. For example, in an embodiment of the present disclosure, the AR processing module 150 may obtain point information in a 3D space from a depth image or may extract RGB feature points, and then may analyze the movement between frames from the point information or the RGB feature points to determine the location and direction of the depth camera.

Each of the feature extraction module 120, the image registration module 130, the image processing module 140, and the AR processing module 150 may be implemented as an algorithm for controlling operations or a program for reproducing the algorithm. In this case, each program may be implemented on a computer by using a memory that stores each program, and at least one processor (not shown) that performs the above-described operations by using data stored in the memory. At this time, the memory and the processor may be implemented as separate chips. Alternatively, the memory and the processor may be implemented as a single chip.

The processor is configured to control overall organic operations of various functional units of an electronic device 100, such as processing one or more commands required for controlling the medical image processing device 10, performing operations according to the commands, and making determination according to program logic. The processor may provide or process appropriate information or functions to a user, by processing signals, data, information, or the like, which is input or output through the input/output I/F 110 or the various processing modules 120 to 150, or driving an application program stored in the database 170. The processed data may be stored in the memory, may be used to build the database 170, or may be transmitted to the outside through the input/output I/F 110 or the various processing modules 120 to 150. The processor may be implemented with a general-purpose processor, a special-purpose processor, or an application processor. In an embodiment, the processor may be implemented as an operation processor (e.g., a central processing unit (CPU), a graphic processing unit (GPU), an application processor (AP), and the like) including dedicated logic circuits (e.g., a field programmable gate array (FPGA), an application specific integrated circuits (ASICs), and the like), but is not limited thereto. In an embodiment, it is not excluded that the processor is implemented as a digital signal processor (DSP) that performs high-speed processing by converting analog signals into digital signals, a micro controller unit (MCU), or a neural processing unit (NPU) specialized in processing an artificial neural network.

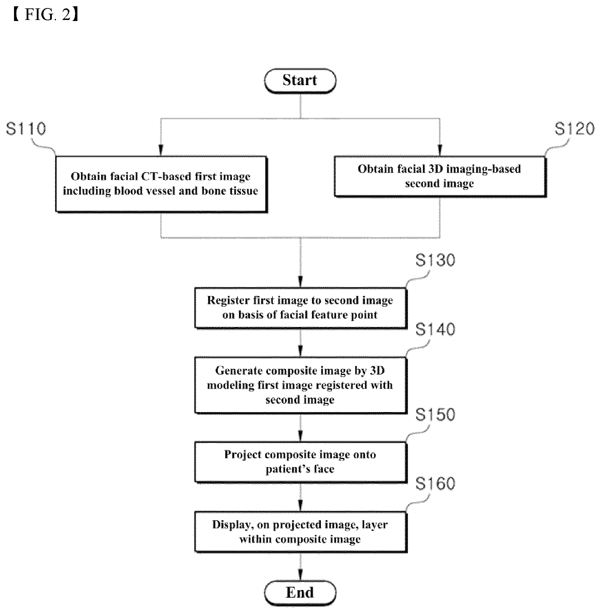

Furthermore, the processor may control one of the components described above or the combination of the components to implement various embodiments of the present disclosure described below with reference to FIGS. 2 to 7 on the present device.

The output I/F 160 may include at least one of a display unit, a sound output interface, a haptic module, and an optical output interface, which are used to generate an output associated with visual, auditory, or tactile sensation. The display unit may have a mutual layer structure with a touch sensor or may be integrally formed with the touch sensor, thereby implementing the touch screen. Such the touch screen may provide an output interface between the present device and a user as well as operating as a user input unit that provides an input interface between the present device and the user.

The display unit displays (outputs) information processed by the present device. For example, the display unit may display execution screen information of an application program (e.g., an application) running on the present device, or a user interface (UI) or graphical user interface (GUI) information according to such the execution screen information.

The sound output interface may output audio data, which is received through a communication unit or stored in the memory, or may output audio signals related to functions performed by the present device. The sound output I/F 160 may include a receiver, a speaker, a buzzer, and the like.

The haptic module generates various tactile effects that the user is capable of feeling. A representative example of the tactile effect generated by the haptic module is vibration. The intensity and pattern of the vibration generated from the haptic module may be controlled by the user's selection or the settings of a control unit. Furthermore, in addition to the vibration, the haptic module may generate various tactile effects such as an effect of stimulation of a pin arrangement that vertically moves on a contact skin surface, blowing force or suction force of air through a nozzle or suction port, rubbing against a skin surface, a contact of an electrode, electrostatic force, or the like, or an effect by the coolness and warmth generated by using elements capable of absorbing heat or generating heat.

The haptic module may not only deliver a tactile effect through a direct contact, and may but also be implemented such that the user is capable of feeling a tactile effect through a muscle sense of a finger or arm. The two or more haptic modules may be provided depending on a configuration aspect of the present device.

The optical output I/F 160 may output a signal for providing a notification that an event occurs, by using light from a light source of the present device, or may output media such as images to be displayed on an external screen by using a projector. In an embodiment, the optical output I/F 160 may adjust the wavelength, magnitude, and projection location of light such that an image is projected three-dimensionally onto an object to express the image in augmented reality.

The output I/F 160 serves as a passage for various types of external devices connected to the present device. The interface unit may include at least one of a wired/wireless headset port, an external charger port, a wired/wireless data port, a memory card port, a port for connecting a device equipped with a subscriber identification module (SIM), an audio input/output (I/O) port, a video I/O port, and an earphone port.

The database 170 refers to a set of data that is collectively managed for the purpose of being shared and used by storing various types of information. The database 170 may store data temporarily or semi-permanently. For example, the database 170 may store an operating system (OS) for operating at least one device, data for hosting a website, or data regarding an application (e.g., a web application). Moreover, the database may store modules in the form of computer code as described above. The database 170 is managed through middleware separate from an application program.

The database 170 includes a relational database (RDB), a key-value database, an object database, a document database, a memory database, and the like.

The database 170 may store data for supporting various functions of the present device, and a program for operations of the control unit, may store pieces of input/output data (e.g., music files, still images, videos, and the like), and may store a plurality of application programs (or applications) running on the present device, pieces of data for operations of the present device, and instructions. At least part of the application programs may be downloaded from an external server through wireless communication.

Furthermore, the database 170 may be separate from the present device, or may be implemented as a memory database connected by wire or wirelessly. In the case, the memory may include the type of a storage medium of at least one of a flash memory type, a hard disk type, a Solid State Disk (SSD) type, a Silicon Disk Drive (SDD) type, a multimedia card micro type, a memory of a card type (e.g., SD memory, XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc.

FIG. 2 is a flowchart illustrating an operating method of the medical image processing device 10 in FIG. 1, according to an embodiment of the present disclosure.

An AR-based medical image processing method according to an embodiment illustrated in FIG. 2 includes steps that are processed in a time-series or parallel manner in the medical image processing device 10 illustrated in FIG. 1, and is referred to as an "operating method of the medical image processing device".

In step S110, the medical image processing device 10 may obtain a first image including blood vessel information and bone tissue information according to tomography from a first external device. In an embodiment, the first external device may be the tomography device 20 (in FIG. 1). In the present disclosure, CT imaging is described by means of example for convenience of description. However, the tomography device 20 may refer to capturing images of the inside of the human body through computed tomography (CT), magnetic resonance imaging (MRI), and positron emission tomography (PET).

According to an embodiment of the present disclosure, the medical image processing device 10 may extract a first facial feature point from the first image. The first facial feature point may be derived from the blood vessel information including information on the location, type, size, and distribution degree of blood vessels distributed in a facial area, and bone tissue information including the location, type, size, and distribution degree of bone tissue constituting the facial area.

In step S120, the medical image processing device 10 may obtain a second image including a facial structure and a facial curve according to 3D imaging from a second external device. According to an embodiment, the 3D imaging device 30 may be a stereo camera and/or a depth camera, and the medical image processing device 10 may obtain 3D image information about an object by using the shooting results of a stereo camera and/or a depth camera.

According to an embodiment of the present disclosure, the medical image processing device 10 may extract a second facial feature point from the second image. The second facial feature point may include the facial curve and the facial structure making up the facial area, and may be derived from the shape and structure of a bone, as well as the result distribution of the skin covering the bone, subcutaneous fat and muscle, and a skin condition including the epidermis and dermis.

In step S130, the medical image processing device 10 may register the first image to the second image based on facial feature points. According to an embodiment, the medical image processing device 10 may extract the facial feature points based on a first facial feature point and a second facial feature point. According to an embodiment, the medical image processing device 10 may extract the facial feature points from facial unique information including the blood vessel information, the bone tissue information, the facial structure, and the facial curve. According to an embodiment, the medical image processing device 10 may register the first image to the second image based on the facial feature points.

In an embodiment, the medical image processing device 10 may perform registration by using a first registration algorithm and a second registration algorithm. For example, On the basis of the first registration algorithm, the medical image processing device 10 may calculate approximate locations, which mean the locations and directions of a first feature point from the first image including a blood vessel and a bone tissue based on facial CT, and a second feature point from a second image including eyes of a face, a nose of the face, a mouth of the face, ears of the face, the shape and skeleton of a facial bone, and a facial structure based on facial 3D imaging. In an embodiment, the medical image processing device 10 may perform downsize-sampling on feature points by using a point pair feature (PPF) algorithm, may select and quantize a point pair, and may calculate approximate locations based on the similarity of the identical and similar point pair in a lookup table. In an embodiment, the medical image processing device 10 may calculate precise locations of a plurality of first feature points and a plurality of second feature points by using an ICP algorithm.

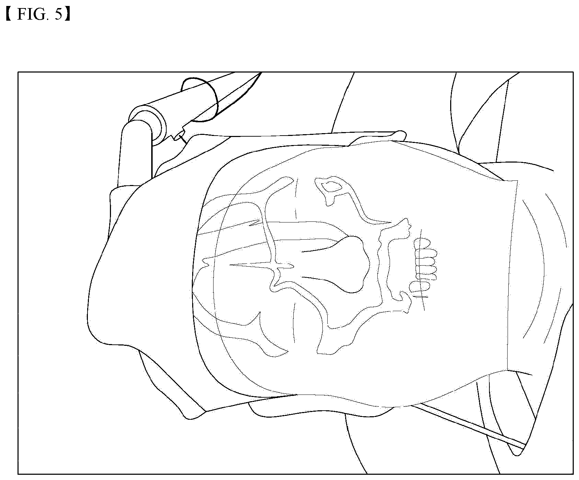

In step S140, the medical image processing device 10 may generate a composite image by performing 3D-modeling on the first image registered with the second image. In an embodiment, the medical image processing device 10 may generate a composite image, which is a target to be projected on the patient's actual face in augmented reality, as a result of registering the first image based on tomography and the second image based on 3D imaging. The image processing results according to step S130 and step S140 will be described in more detail with reference to FIG. 5.

In step S150, the medical image processing device 10 may project the composite image onto the patient's face. According to an embodiment, the medical image processing device 10 may obtain a real-time patient facial image from an external device. According to an embodiment, the medical image processing device 10 may search for facial feature points from the real-time patient facial image. According to an embodiment, the medical image processing device 10 may continuously calculate the registration between the composite image and the facial feature points depending on the change in the real-time patient facial image.

According to an embodiment, in a step for continuously calculating the registration between the composite image and the facial feature points depending on the change in the real-time patient facial image, the medical image processing device 10 may perform primary registration on the composite image and the real-time patient facial image based on a portion corresponding to locations of eyes.

According to an embodiment, the medical image processing device 10 may perform the primary registration, and then may perform second registration on the composite image and the real-time patient face image based on portions corresponding to the face other than the locations of eyes.

In an embodiment, the medical image processing device 10 may perform primary registration on the composite image with the real-time patient face image based on portions corresponding to the locations of eyes. In an embodiment, the image registration module 130 may perform the primary registration to increase the accuracy of registration, and then perform second registration on the composite image and the real-time patient face image based on portions corresponding to the face other than the locations of eyes. That is, the image registration module 130 may increase the accuracy of registration by first performing primary registration based on information about reference points (e.g., both eyes) relatively less likely to be deformed.

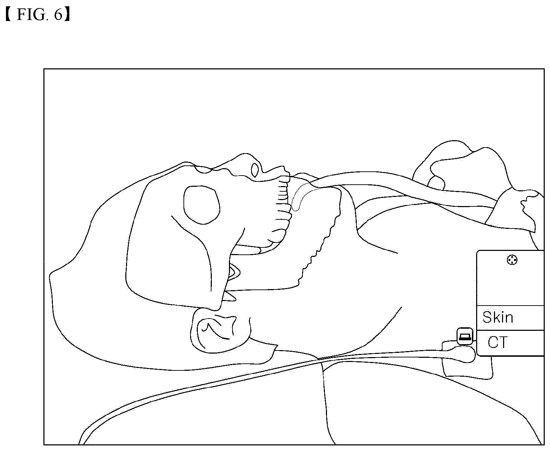

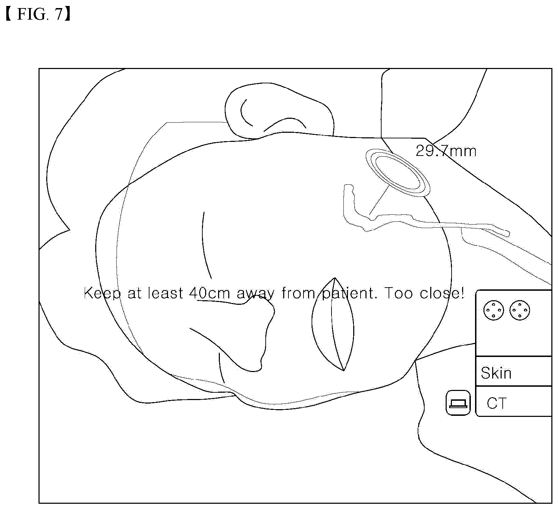

In step S160, the medical image processing device 10 may display a first layer corresponding to blood vessel information, and a second layer including bone tissue information on a projected image. According to an embodiment, the medical image processing device 10 may display the first layer. According to an embodiment, the medical image processing device 10 may display the second layer separately at a point in time when the first layer is not displayed, or in an area where it is not displayed. Step S150 and step S160 will be described in more detail with reference to FIGS. 6 and 7.

At least one component may be added or deleted to correspond to the performance of the components illustrated in FIG. 2. Furthermore, it will be easily understood by those skilled in the art that mutual locations of the components may be changed to correspond to the performance or structure of the system.

In the meantime, each component shown in FIG. 2 refers to software components and/or hardware components such as field programmable gate array (FPGA) and application specific integrated circuit (ASIC).

According to the above-mentioned problem solving means of the present disclosure, a facial AR technology may be provided to minimize errors in consideration of the complex and delicate structure of a facial area through AR technology centered on the facial area.

According to the above-mentioned problem solving means of the present disclosure, a device, a method, and a computer program for processing a medical image based on augmented reality that may improve the accuracy of coordinate system registration through non-marker-based coordinate system registration may be provided.

According to the above-mentioned problem solving means of the present disclosure, the occurrence of human error may be suppressed in all processes of using augmented reality, either while there is no need to install markers, or while relative locations of markers to a patient's body are maintained after markers are installed.

According to the present disclosure, an image registered with a CT image in the 3D image may be projected onto a patient's facial area, and thus the location and depth of each of a bone tissue and a blood vessel tissue placed within the facial area may be visually displayed, thereby achieving the accurate and safe surgery of the facial area.

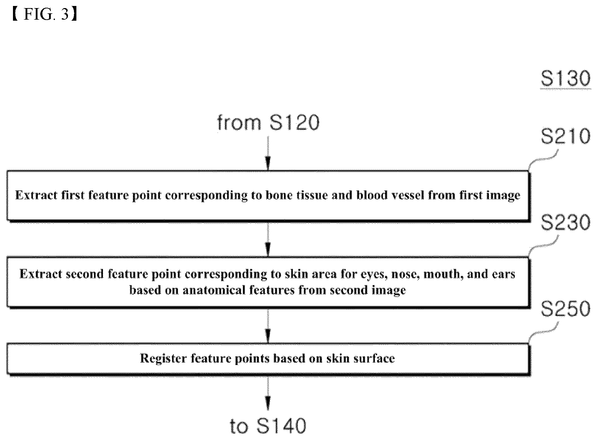

FIG. 3 is a detailed flowchart of step S130 of an operating method of the medical image processing device 10 (in FIG. 1), according to an embodiment of the present disclosure.

After performing step S120, in step S210, the medical image processing device 10 may extract at least one first feature point corresponding to a bone tissue and a blood vessel from a first image. According to an embodiment, the medical image processing device 10 may extract a first feature point from the first image including blood vessel information and bone tissue information according to tomography from an external device.

In step S230, the medical image processing device 10 may extract at least one second feature point corresponding to a skin area for the eyes, nose, mouth, and ears of a human body based on anatomical features. According to an embodiment, the medical image processing device 10 may obtain a second image including a facial structure and a facial curve according to 3D imaging from an external device, and the 3D imaging device 30 may be a stereo camera and/or a depth camera. The medical image processing device 10 may derive at least one second feature point including a skull bone, a nasal bone, an earlobe, and a jaw bone based on anatomical features from the result of obtaining 3D image information about an object by using the shooting results of a stereo camera and/or a depth camera. The skull bone, the nasal bone, the earlobe, and the jaw bone are elements capable of determining the facial curve and facial structure, and the second feature point may be derived from the shape and structure of a bone, as well as the result distribution of the skin covering the bone, subcutaneous fat and muscle, and a skin condition including the epidermis and dermis. The second feature point may be derived from the characteristics of an image, or may be extracted as data resulting from a hidden layer through feature extraction of a vision-based artificial intelligence learning model (e.g., Convolutional Neural Network (CNN)).

According to an embodiment of the present disclosure, the medical image processing device 10 may extract a second feature point from the first image. The second feature point may be derived from the blood vessel information including information on the location, type, size, and distribution degree of blood vessels distributed in a facial area, and bone tissue information including the location, type, size, and distribution degree of bone tissue constituting the facial area.

**In** step S250, the medical image processing device 10 may extract facial feature points based on first feature points and second feature points. According to an embodiment, the medical image processing device 10 may register the facial feature points based on a skin surface derived from the second image. According to an embodiment, the medical image processing device 10 may calculate an approximate location of at least one second feature point including a skull bone, a nose bone, an earlobe, and a jaw bone and at least one first feature point including a bone tissue and a blood vessel based on anatomical features based on a first registration algorithm. According to an embodiment, the medical image processing device 10 may calculate a precise location of the at least one first feature point and the at least one second feature point based on a second registration algorithm. The first and second registration algorithms have been described in detail with reference to FIGS. 1 and 2, and thus redundant descriptions are omitted.

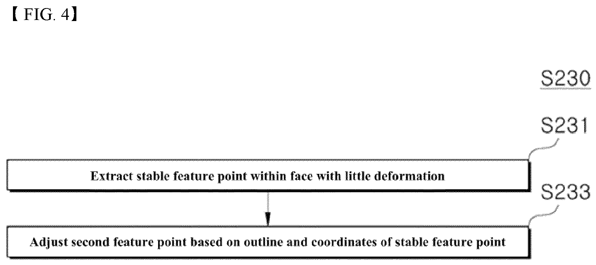

FIG. 4 is a detailed flowchart of step S230 of an operating method of a medical image processing device, according to an embodiment of the present disclosure.

In step S231, the medical image processing device 10 may extract a stable feature point within a face with little deformation. According to an embodiment, the patient is under anesthesia during a surgery or a procedure, and thus a change is not great. However, depending on the surgery or procedure performed by the medical staff, the location and the angle may be slightly changed as the facial area is touched by hands or medical instruments. The medical image processing device 10 may set eyes, which have little deformation and easy search even when movement or fluctuation occurs, as reference points and may extract a stable feature point from locations of the eyes and the size ratio with the entire face. In the present disclosure, only the eyes are illustrated for convenience of description. However, various tissues, blood vessels, muscles, structures, or the like within a body, in which easy search is possible and changes or fluctuations are not great when observed from above a face may be used as stable feature points.

In step S233, the medical image processing device 10 may adjust a second feature point based on an outline and coordinates of the stable feature point. According to an embodiment, the stable feature point may be the location and size of eyes with respect to the entire facial contour.

FIG. 5 is a diagram illustrating the synthesis of a tomography image and a 3D image according to an image registration operation of a medical image processing device, according to an embodiment of the present disclosure.

Referring to FIG. 2 together, in step S130, the medical image processing device 10 may register a first image to a second image based on facial feature points. According to an embodiment, the medical image processing device 10 may extract the facial feature points from facial unique information including the blood vessel information, the bone tissue information, the facial structure, and the facial curve. According to an embodiment, the medical image processing device 10 may register the first image to the second image based on the facial feature points. In step S140, the medical image processing device 10 may generate a composite image by performing 3D-modeling on the first image registered with the second image. In step S150, the medical image processing device 10 may project the composite image onto the patient's face.

In an embodiment, the medical image processing device 10 may obtain a real-time patient facial image from an external device and may search for facial feature points from the real-time patient facial image. The medical image processing device 10 may continuously calculate the registration between the composite image and the facial feature points depending on the change in the real-time patient facial image.

In an embodiment, the medical image processing device 10 may perform primary registration between the composite image and the real-time patient facial image based on a portion corresponding to locations of eyes, and may perform secondary registration between the composite image and the real-time patient facial image based on a portion corresponding to the face other than the locations of eyes.

In an embodiment, the medical image processing device 10 may project the composite image onto the patient's actual facial area in augmented reality by using a simultaneous localization and mapping (SLAM) algorithm.

FIG. 6 is a diagram showing a projection of a bone tissue layer according to an image synthesis operation of the medical image processing device 10, according to an embodiment of the present disclosure. FIG. 7 is a diagram showing a projection of a blood vessel layer according to an image synthesis operation of a medical image processing device, according to an embodiment of the present disclosure.

In a step of displaying a first layer corresponding to blood vessel information and a second layer including bone tissue information on a projected image, the medical image processing device 10 may display the first layer. In an embodiment, the medical image processing device 10 may display the second layer separately at a point in time when the first layer is not displayed, or in an area where it is not displayed. In an embodiment, the medical image processing device 10 may obtain a real-time patient facial image from a second external device.

Referring to FIG. 6, the medical image processing device 10 may project a facial area skeleton constituting a bone tissue and/or a facial structure captured from a patient onto a location corresponding to the patient's actual face by displaying the second layer.

Referring to FIG. 7, the medical image processing device 10 may project an actual blood vessel tissue captured from the patient onto a location corresponding to the patient's actual face by displaying the first layer.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. An operating method of a medical image processing device using augmented reality (AR), the method comprising:
obtaining, from a first external device, a first image including blood vessel information and bone tissue information according to tomography;
obtaining, from a second external device, a second image including a facial structure and a facial curve according to 3D imaging;
extracting a facial feature point from facial unique information including the blood vessel information, the bone tissue information, the facial structure, and the facial curve;
registering the first image to the second image based on the facial feature point;
generating a composite image by performing 3D-modeling on the first image registered with the second image;
projecting the composite image onto a face of a patient; and
displaying, on a projected image, a first layer corresponding to the blood vessel information and a second layer including the bone tissue information.

2. The method of claim 1, wherein the extracting of the facial feature point from the facial unique information includes:
extracting at least one first feature point corresponding to a bone tissue and a blood vessel from the first image;
extracting at least one second feature point corresponding to a skin area for eyes, a nose, a mouth, and ears of a human body based on an anatomical feature from the second image; and
extracting the facial feature point based on the first feature point and the second feature point.

3. The method of claim 2, wherein the registering of the first image to the second image based on the facial feature point includes:
registering the facial feature point based on a skin surface derived from the second image.

4. The method of claim 2, wherein the extracting of the at least one second feature point includes:
extracting a stable feature point within a face with little deformation; and
adjusting the second feature point based on an outline and coordinates of the stable feature point, and
wherein the stable feature point is a location and a size of eyes with respect to an entire facial contour.

5. The method of claim 1, wherein the facial feature point serves as a marker used in image registration.

6. The method of claim 1, wherein the projecting of the composite image onto the face of the patient includes:
obtaining a real-time patient facial image from the second external device;
searching for the facial feature point from the real-time patient facial image; and
continuously calculating registration between the composite image and the facial feature point depending on a change in the real-time patient facial image, and
wherein the calculating of the registration between the composite image and the facial feature point depending on the change in the real-time patient facial image continuously includes;
performing primary registration on the composite image and the real-time patient facial image based on a portion corresponding to locations of eyes; and
after performing the primary registration, performing secondary registration on the composite image and the real-time patient facial image based on a portion corresponding to a face other than the locations of the eyes.

7. The method of claim 1, wherein the registering of the first image to the second image based on the facial feature point includes:
calculating approximate locations of at least one first feature point corresponding to a bone tissue and a blood vessel, and at least one second feature point corresponding to a skin area for eyes, a nose, a mouth, and ears of a human body from the second image based on a first registration algorithm; and
calculating precise locations of the at least one first feature point and the at least one second feature point based on a second registration algorithm.

8. The method of claim 1, wherein the displaying of the first layer corresponding to the blood vessel information and the second layer including the bone tissue information on the projected image includes:
displaying the first layer; and
separately displaying the second layer at a point in time when the first layer is not displayed, or in a region where the first layer is not displayed.

9. A medical image processing device using AR comprising:
a memory configured to store a feature extraction module configured to extract a feature part from an image, an image registration module configured to register at least two images, an image processing module configured to perform image processing on data constituting an image according to a predefined operation, and an AR processing module configured to implement and display the processed image in augmented reality; and
a processor including one or more cores and configured to control operations of the feature extraction module, the image registration module, the image processing module, and the AR processing module,
wherein the memory is configured to:
obtain a first image including blood vessel information and bone tissue information, and a second image including a facial structure and a facial curve from an external device, and
wherein the processor is configured to:
extract a facial feature point from facial unique information including the blood vessel information, the bone tissue information, the facial structure, and the facial curve;
register the first image to the second image based on the facial feature point;
generate a composite image by performing 3D-modeling on the first image registered with the second image;
project the composite image onto a face of a patient; and
display, on a projected image, a first layer corresponding to the blood vessel information and a second layer including the bone tissue information.

10. The medical image processing device of claim 9, wherein when extracting the facial feature point, the processor is configured to:
extract at least one first feature point corresponding to a bone tissue and a blood vessel from the first image;
extract at least one second feature point corresponding to a skin area for eyes, a nose, a mouth, and ears of a human body based on an anatomical feature from the second image; and
extract the facial feature point based on the first feature point and the second feature point.

11. The medical image processing device of claim 10, wherein when registering the first image to the second image based on the facial feature point, the processor is configured to:
register the facial feature point based on a skin surface derived from the second image.

12. The medical image processing device of claim 10, wherein when extracting the at least one second feature point, the processor is configured to:
extract a stable feature point within a face with little deformation; and
adjust the second feature point based on an outline and coordinates of the stable feature point, and
wherein the stable feature point is a location and a size of eyes with respect to an entire facial contour.

13. The medical image processing device of claim 9, wherein the facial feature point serves as a marker used in image registration.

14. The medical image processing device of claim 9, wherein the memory is further configured to:
obtain a real-time patient facial image from the external device, and
wherein when projecting the composite image onto the face of the patient, the processor is configured to:
search for the facial feature point from the real-time patient facial image; and
continuously calculate registration between the composite image and the facial feature point depending on a change in the real-time patient facial image.

15. The medical image processing device of claim 9, wherein when registering the first image to the second image, the processor is configured to:
calculate approximate locations of at least one first feature point corresponding to a bone tissue and a blood vessel, and at least one second feature point corresponding to a skin area for eyes, a nose, a mouth, and ears of a human body from the second image based on a first registration algorithm; and
calculate precise locations of the at least one first feature point and the at least one second feature point based on a second registration algorithm.
